Europäisches Patentamt

European Patent Office

Office européen des brevets

(19)

(11) Numéro de publication: **0 212 680**
**A1**

(12) **DEMANDE DE BREVET EUROPEEN**

(21) Numéro de dépôt: 86200118.7

(22) Date de dépôt: 29.01.86

(51) Int. Cl.⁴: **A61L 9/12** , A63H 3/02 ,
A61L 9/03 , //A61K35/78

(30) Priorité: 18.07.85 BE 6048119

(43) Date de publication de la demande:
04.03.87 Bulletin 87/10

(84) Etats contractants désignés:
AT CH DE FR GB IT LI LU NL SE

(71) Demandeur: **Gilliquet, Robert**
**9124 Collins Avenue, Suite 205**
**Miami Florida 33154(US)**

(72) Inventeur: **Gilliquet, Robert**
**9124 Collins Avenue, Suite 205**
**Miami Florida 33154(US)**

(74) Mandataire: **Dellicour, Paul**
**Office de Brevets E. Dellicour rue Fabry**
**18/012**
**B-4000 Liège(BE)**

(54) **Procédé de mise en oeuvre de plantes médicinales.**

(57) Le procédé consiste à enfermer la substance à mettre en oeuvre dans une enveloppe destinée à subir un échauffement. La substance est enfermée en vrac dans un objet en peluche ou autre matière appropriée ou au préalable dans un sac ou sachet.

EP 0 212 680 A1

## Procédé de mise en oeuvre de plantes médicinales

La présente invention concerne les herbes ou plantes médicinales et a pour objet un procédé pour dégager l'arôme de ces substances d'origine végétale.

On sait en effet que de certaines herbes ou plantes médicinales s'échappe un principe odorant qui a une action bienfaisante notamment sur les voies respiratoires.

C'est pourquoi il a paru avantageux de créer un procédé de mise en oeuvre de ces herbes ou plantes médicinales. Le procédé est caractérisé en ce qu'il consiste à enfermer la substance à mettre en oeuvre dans une enveloppe destinée à subir un échauffement.

Suivant l'invention le procédé consiste à incorporer la substance, herbes ou plantes médicinales, dans un coussin ou dans un élément, tel qu'une poupée ou un animal, en tissu approprié pour permettre le dégagement de l'arôme lorsqu'il est au contact d'une source de chaleur.

Suivant l'invention la substance est avantageusement renfermée au préalable dans un sac ou sachet en matière appropriée.

Avec le procédé suivant l'invention on incorpore, par exemple, dans des jouets en peluche, des poupées ou des animaux en peluche ou en une autre matière appropriée des herbes aromatiques naturelles sèches, sans additif chimique, des herbes médicinales ou analogues.

Ces herbes peuvent être au préalable enveloppées de matière textile, de papier ou de toute autre matière appropriée permettant aux herbes d'en laisser dégager leur arôme. Cette enveloppe peut se présenter sous forme de sac ou sachet.

Le jouet, la poupée ou l'animal ou encore un simple coussin renfermant les herbes médicinales en vrac ou en sac ou sachet, au contact d'une source de chaleur, humaine par exemple, va permettre le dégagement de l'arôme desdites herbes et ainsi remédier aux problèmes des voies respiratoires de l'enfant ou de la personne qui utilise l'objet en question.

## Revendications

1. Procédé de mise en oeuvre de plantes médicinales, caractérisé en ce qu'il consiste à enfermer la substance à mettre en oeuvre dans une enveloppe destinée à subir un échauffement.

2. Procédé suivant la revendication 1, caractérisé en ce qu'il consiste à enfermer la substance en vrac dans un coussin ou un objet en une matière appropriée permettant aux plantes d'en laisser dégager leur arôme.

3. Procédé suivant la revendication 1, caractérisé en ce qu'il consiste au préalable à enfermer la substance dans un sac ou sachet en une matière appropriée permettant aux plantes d'en laisser dégager leur arôme et ensuite à incorporer ledit sac ou sachet dans un coussin ou objet en matière appropriée.

4. Coussins ou jouets, tel que poupées et animaux, en peluche ou autre matière appropriée, renfermant des herbes ou plantes médicinales pour réaliser le procédé suivant l'invention.

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl.4) |
|---|---|---|---|
| Y | FR-A-1 041 075  (SIPAR) <br> * Page 1, lignes 36-40; résumé * <br><br> --- | 1-3 | A 61 L   9/12 <br> A 63 H   3/02 <br> A 61 L   9/03  // <br> A 61 K  35/78 |
| Y | GB-A-  735 687  (J.A. PAREDES NOGUERA) <br> * Page 1, lignes 9-16 * <br><br> ----- | 1-3 | |

DOMAINES TECHNIQUES
RECHERCHES (Int. Cl.4)

A 61 L
A 63 H

Le present rapport de recherche a été établi pour toutes les revendications

| Lieu de la recherche <br> LA HAYE | Date d'achèvement de la recherche <br> 30-10-1986 | Examinateur <br> PELTRE CHR. |
|---|---|---|

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

& : membre de la même famille, document correspondant

OEB Form 1503 03 82